# EUROPEAN PATENT SPECIFICATION

(11) **EP 0 556 482 B1**
(45) Date of publication and mention of the grant of the patent: **15.05.1996**
(21) Application number: 92121791.5
(22) Date of filing: 22.12.1992
(51) Int. Cl.: C07K 7/64, A61K 38/55, A61K 38/57

(54) **Propyl endopeptidase inhibitor**
Prolyl Endopeptidase Inhibitor
Inhibiteur de la "prolyl endopeptidase"

(30) Priority: 24.12.1991 JP 357004/91
(43) Date of publication of application: 25.08.1993
(73) Proprietor: SNOW BRAND MILK PRODUCTS CO., LTD., Sapporo-shi, Hokkaido 065 (JP)
(72) Inventor: Kimura, Kenichi, Utsunomiya-shi, Tochigi (JP); Nakamura, Junji, Ishibashimachi, Shimotsuga-shi, Tochigi (JP); Kanou, Fumiko, Utsunomiya-shi, Tochigi (JP); Yoshihama, Makoto, Utsunomiya-shi, Tochigi (JP)
(74) Representative: Boeters, Hans Dietrich, Dr.

(56) References cited:
- J ANTIBIOT vol. 17, no. 1, January 1989, pages 107 - 115 TAKE Y., ET AL. 'Comperative studies of the inhibitory properties of antibiotics on human immunodeficiency virus reverse transcriptases and cellular DNA polymerases'
- J ANTIBIOT vol. 15, no. 1, January 1987, pages 100 - 104 INOUYE Y., ET AL. 'Screening for inhibitors of avian myeloblastosis virus reverse transcriptase and effect on the replication of AIDS-virus'

## Description

### BACKGROUND OF THE INVENTION

### Field of the Invention

The present invention relates to a prolyl endopeptidase inhibitor. The prolyl endopeptidase inhibitor of the present invention is effective for curing amnesia due to its possession of a prolyl endopeptidase inhibitive activity.

### Description of the Background Art

Along with a prolonged life span brought about by the development of medical technologies in recent years, senile dementia has become a serious problem not only for the patients himself, but also for the family and society. Drugs heretofore proposed and used for the cure of dementia have been those exhibiting their actions on the marginal factors of dementia. A drug for providing its action on the essence of dementia itself has been desired.

Prolyl endopeptidase (EC 3.4.21.26) is an enzyme capable of specifically cutting the carboxyl side of proline in proline-containing peptides, both *in vitro* and *in vivo*. The enzyme has an activity of decomposing or inactivating nerve peptide vasopressin which is considered to be associated with memory [Nippon Nôgeikagaku kaishi, 58, 1147-1154 (1984)]. Prolyl endopeptidase inhibitors are therefore expected to have an antiamnesia activity. In fact, a number of synthetic inhibitors have been reported to exhibit an antiamnesia activity in experiments using rats and mice [J. Pharmacobio-Dyn, 10, 730-735 (1987)].

Furthermore, several reports have recently been published regarding relationship between Alzheimer type dementia and prolyl endopeptidase; e.g., a report dealing with a significant increase in the activity of this enzyme in brains of Alzheimer patients [Experientia, 46, 94-97 (1990)], a report about the involvement of the enzyme in β-protein C-terminal cutting off [FEBS LETTERS, 260, 131-134 (1990)], and the like. Prolyl endopeptidase inhibitors are thus enthusiastically expected to be useful as an antiamnesia drug. In particular, most of conventional inhibitors are synthetic drugs with similar chemical structures. Structural activity correlations for synthetic inhibitors have been elucidated [Agric. Biol. Chem., 55, 37-43 (1991)], and thus their activities, absorption, excretion, stability, and the like are being clarified in animal experiments. In this regard, inhibitors naturally produced by actinomycetes and the like are expected to possess unique and complicated structures which are not found in synthetic inhibitors. There are expectations that they have advantages which have not been possessed by chemical inhibitors in terms of their activities, absorption, excretion, stability, and the like in vivo,

Further, J. Antibiot., 15 (1987) 100-104 and 17 (1989) 107-115 disclose an inhibitory effect of Enduracidin A on reverse transcriptase which might be of potential therapeutic importance for combating AIDS.

### SUMMARY OF THE INVENTION

The present inventors have undertaken studies for obtaining inhibitors having prolyl endopeptidase inhibitive activity from natural sources and discovered that enduracidin A and its analogs, which have conventionally been known to exhibit antimicrobial activities, unexpectedly also possess prolyl endopeptidase inhibitive activity, and found that this activity can be utilized for the cure of amnesia.

Enduracidin A and its analogs of which the prolyl endopeptidase inhibitive activity was found by the present inventors are known compounds. Their possession of the prolyl endopeptidase inhibitive activity has been found for the first time by the present inventors. In addition, these compounds were found to exhibit a stronger action to prolyl endopeptidase derived from human than that derived from microorganism. They were found to have different, more advantageous characteristics as a medicine than known inhibitors.

Specifically, an object of the present invention is to provide a novel prolyl endopeptidase inhibitor which exhibits more advantageous characteristics than known drugs in terms of its activity, absorption, excretion, safety, and the like.

A further object of the present invention is to provide a drug composition for curing amnesia, in which the prolyl endopeptidase inhibitive activity is utilized.

The above object can be achieved according to the present invention by the provision of a method of inhibiting prolyl endopeptidase activity comprising administering to a subject requiring the inhibition of prolyl endopeptidase an effective amount of the following compound (I), 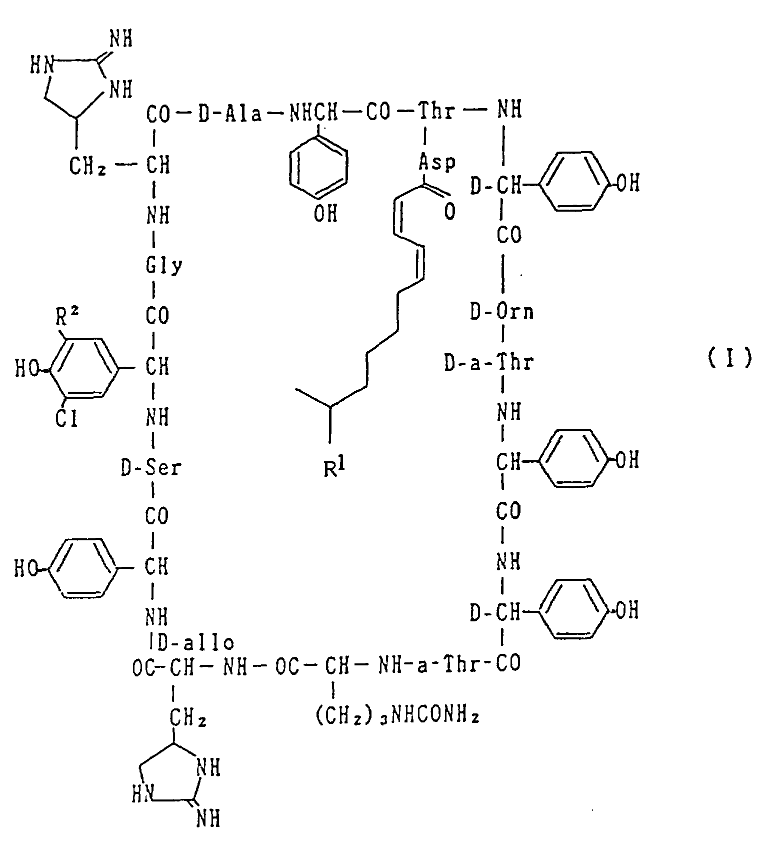 wherein R¹ represents an alkyl group and especially a lower alkyl group, R is a halogen atom or a hydrogen atom, Ala stands for an alanine residue, Thr a threonine residue, Asp an aspartic acid residue, Orn an ornithine residue, Ser a serine residue, Gly a glycine residue, D stands for D-type, allo indicates a stereo isomer, and "a" indicates allo; or a pharmaceutically acceptable salt thereof.

In a preferred embodiment of the present invention, said compound of formula (I) is enduracidin A.

The above object can be further achieved according to the present invention by the provision of a method of curing amnesia, comprising administering to a subject an effective amount of said compound (I) or a pharmaceutically acceptable salt thereof.

### BRIEF DESCRIPTION OF THE DRAWINGS

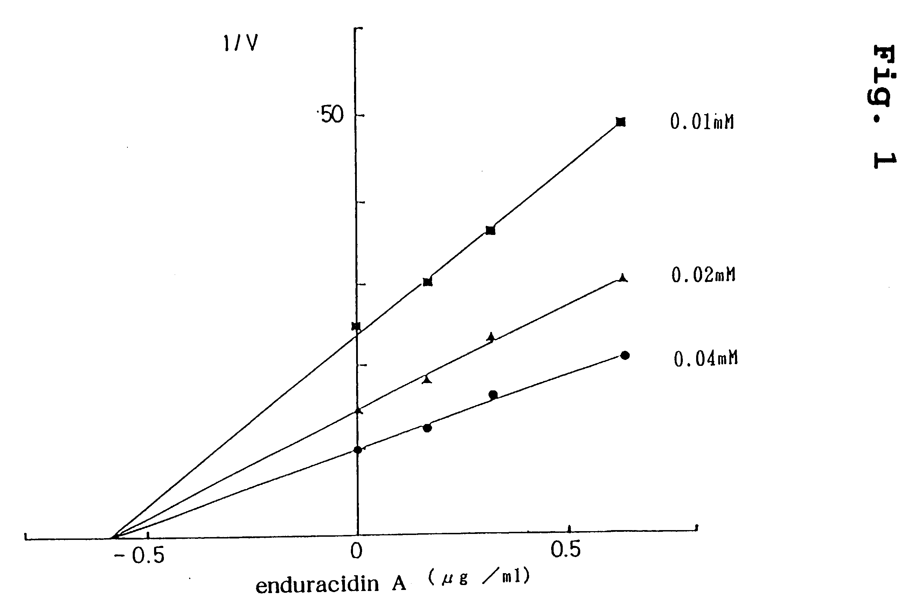

Figure 1 is a chart showing the relationship between dose of enduracidin A and the prolyl endopeptidase inhibitive activity by Dixon Plot.

### DETAILED DESCRIPTION OF THE INVENTION AND PREFERRED EMBODIMENTS

The lower alkyl group represented by R¹ in formula (I) may be methyl, ethyl, propyl, isobutyl, sec-butyl, n-butyl or tert-butyl group, or the like; and the halogen atom represented by R may be chlorine, bromine, or fluorine.

The effective component of the present invention may form a pharmaceutically acceptable salt, such as hydrochloride, phosphate, or the like. Thus, the effective component in the present invention includes such pharmaceutically acceptable salts of compound of formula (I).

The compound having a methyl group for R¹ and a chlorine atom for R in formula (I) is enduracidin A which is a known compound.

The enduracidins represented by the above formula (I) and typified by the enduracidin A which are obtained by cultivating a microorganism are reported to be usable as an antimicrobial agent or an additive for animal food due to their antimicrobial activity [Japanese Patent Laid-open (ko-kai) No. 114/1970, Japanese Patent Laid-open (ko-kai) No. 17158/1970, J. Antibiot., 21, 147 (1968), and Reports from J. Takeda Rcs. Lab. 42, 45 (1984)]. These compounds therefore can be obtained by known methods. If possible, they may be prepared by a totally or partially synthetic method.

The enduracidin A and its analogs which are prolyl endopeptidase inhibitive compounds are prepared into tablets, powders, granules, capsules, injections, inhalation agents, or agents for external application according to conventional methods. They can be clinically administered orally or parenterally as an anti-amnesia agent, AIDS prevention or curing agent, or anti-HIV agent. A dose varies depending on symptoms of the subject and the manner in which the drug is administered, although usually an amount of 1 mg to 10 mg per day is administered to an adult once a day or dividedly several times a day.

Enduracidin A is a low toxic compound. Its the acute toxicity LD₅₀ recognized in mouse by intraperitoneal injection and in rat by intravenous injection are reported to be greater than 400 mg/kg and 300 mg/kg, respectively (Japanese Patent Laid-open (ko-kai) No. 114/1970, Japanese Patent Laid-open (ko-kai) No. 17158/1970).

### EXAMPLES

### <Purification Method>

Enduracidins were produced by an antibiotic B-4577-m-producing microorganism belonging to the genus *Streptomyces,* microorganism B-5477 (IFO-12439, ATCC-21013) belonging to *Streptomyces fungicidix,* or other microorganisms which have different characteristics with respect to media and are naturally or artificially mutative. Production, isolation and purification were carried out according to a method described in J. Antibiot., 21, 138 (1968).

### <Preparation of composition>

Hydrochlorides of enduracidins are easily soluble in water and methanol. Thus, they can be used as an injection preparation by dissolving in physiological saline for injection use.

They can also be prepared into tablets together with other components. An example of a formulation which can be made into tablets is given below.

| | |
|---|---|
| Enduracidin A | 200 mg |
| Hydroxypropyl methyl cellulose (HPC) | 30 mg |
| Lactose | 67 mg |
| Magnesium stearate | 3 mg |
| Total | 300 mg |

A tablet preparation containing the above components in amounts indicated above was prepared by blending these components and tabletting the blend.

Enduracidin A which is the compound possessing an activity of inhibiting prolyl endopeptidase in the present invention has the following physiological characteristics.

### 1) Prolyl endopeptidase inhibitive activity

The inhibitive activity of enduracidin A was measured by using a methanol solution of this compound. As a result, the 50% reaction inhibitive concentration for prolyl endopeptidase derived from human placenta was found to be 0.52 µg/ml (0.22 µM) when 0.04 mM Z-Gly-Pro-7AMC was used as a substrate. In the same reaction, the enzyme derived from a microorganism *Flavobacterium* (0.00085 units/ml) exhibited a 50% reaction inhibitive concentration, i.e., 24 µg/ml (10 µM). Thus, enduracidin A was found to exhibit a much higher specificity to the enzyme derived from human placenta (see Table 1 below). On the other hand, SNA-115 which is a naturally occurring compound and Z-pro-prolinol which is a synthetic inhibitor, both known as possessing a prolyl endopeptidase inhibitive activity, exhibited almost the same degree of inhibitive activity to both the enzyme derived from human placenta and the enzyme derived from *Flavobacterium*. Staurosporine, known as inhibiting the enzyme derived from *Flavobacterium* [Agric. Biol. Chem., 54, 3021-3022 (1990)], contrary to enduracidin A, exhibited very weak inhibitive activity to the enzyme derived from human placenta. Thus, the specificity for inhibiting the enzyme derived from human placenta is considered to be a characteristic intrinsic to enduracidin A.

**TABLE 1**

| Inhibitive activity of various compounds for prolyl endopeptidase derived from human placenta and prolyl endopeptidase derived from *Flavobacterium* | | |
|---|---|---|
| Compound | IC₅₀ (µM) | |
| | Human placenta | *Flavobacterium* |
| Enduracidin A | 0.22 | 10.0 |
| SNA-115 | 0.22 | 0.35 |
| Z-Pro-prolinol | 205 | 175 |
| Staurosporine | > 107 | 0.75 |

Reactions of the enzyme from human placenta and enduracidin A were carried out by changing the amount of substrate and the concentration of enduracidin A. The mode of inhibition was investigated by the Dixon Plot to find that enduracidin A exhibited a noncompetitive inhibition with *Ki=*0.6 µg/ml (0.25 µM) (See Figure 1).

The prolyl endopeptidase inhibitive activity in the present invention was measured by the following method. Prolyl endopeptidase from human placenta was prepared according to the method for preparing aromatase [Chem. Pharm. Bull., 38, 2834-2837 (1990)] using cytosol fractions from which microsome fractions (aromatase fractions) have been removed. Specifically, human placenta membrane was removed and the remaining tissues were washed with 1.1% KCl to remove blood therefrom. The placenta tissues were pulverized and homogenized with a 67 mM potassium phosphate buffer (pH 7.5) containing 5 mM DTT and 0.25 M sucrose. The homogenized solution was centrifuged at 10,000 x g for 30 minutes to obtain a supernatant. The supernatant was submitted to ultra centrifugation at 104,000 x g for 60 minutes, thus obtaining a supernatant, from which microsome fractions had been removed, as a prolyl endopeptidase fraction. This crude enzyme was adjusted to a protein concentration of 27.3 mg/ml and pipetted into 1 ml portions and stored at -20°C.

To carry out the enzyme reaction, 0.01 ml of each sample dissolved in methanol in various concentrations and 0.01 ml of the placenta cytosol fraction were added to 0.96 ml of a 0.1 M Tris-HCl buffer solution (pH 7.0), and the resulting solution was mixed and incubated at 37°C for 5 minutes. The reaction was initiated by the addition of 0.02 ml of a 2 mM solution of synthesized substrate, Z-Gly-Pro-7-AMC (a product of Cambridge Research Biochemicals), dissolved in 40% dioxane to said solution, and continued for 10 minutes at 37°C. After the reaction, 0.5 ml of a reaction termination agent (10 g of Triton X-100/95 ml, 1 M acetate buffer, pH 4.0) and 2 ml of water were added to measure 7-AMC (7-amino-4-methylcoumarin) liberated by the reaction at an excitation wavelength of 380 nm and an emission wavelength of 460 nm.

On the other hand, for carrying out the reaction of the enzyme derived from *Flavobacterium,* 0.01 ml of each sample dissolved in methanol in various concentrations and 0.01 ml (0.085 unit/ml) of prolyl endopeptidase (a product of Seikagaku Kogyo Co.) dissolved in 0.1 M Tris-HCl buffer solution (pH 7.0) were added to 0.96 ml of a 0.1 M Tris-HCl buffer solution (pH 7.0), and the resulting solution was mixed and incubated at 37°C for 5 minutes. The reaction was initiated by the addition of 0.02 ml of a 2 mM solution of synthesized substrate, Z-Gly-Pro-7-AMC dissolved in 40% dioxane to said solution, and continued for 10 minutes at 37°C. After the reaction, 0.5 ml of a reaction termination agent (10 g of Triton X-100/95 ml, 1 M acetate buffer, pH 4.0) and 2 ml of water were added to measure 7-AMC liberated by the reaction at an excitation wavelength of 380 nm and an emission wavelength of 460 nm.

The inhibition rate was determined by the formula, [(A-B)/A] x 100 (%), wherein A represents a fluorescent concentration of liberated 7-AMC in the reaction of the control and B is a fluorescent concentration of liberated 7-AMC in the reaction to which a sample was added.

Enduracidin A and its analogs exhibit an inhibitive activity to prolyl endopeptidase derived from human with a higher specificity than conventionally known prolyl endopeptidase inhibitors at a smaller amount. In addition they can noncompetitively act on the enzyme. Thus, they are effective for curing amnesia.

## Claims (Claims for the following Contracting State(s): DE, FR, GB)

1. Use of the following compound (I): 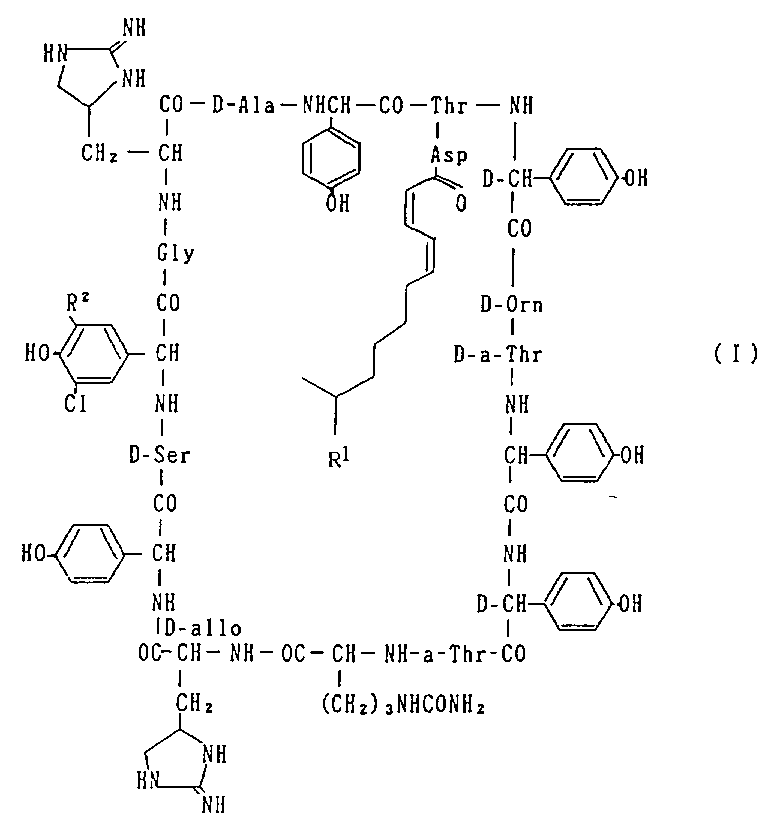 wherein R¹ represents an alkyl group, R is a halogen atom or a hydrogen atom, Ala stands for an alanine residue, Thr a threonine residue, Orn an ornithine residue, Ser a serine residue and Gly a glycine residue, D stands for D-type, and a = allo indicates an stereo isomer; or one of its therapeutically acceptable salts
for the production of a therapeutical agent for an inhibition of prolylendopeptidase activity in a subject.

2. Use of the compound (I) defined in claim 1 or one of its therapeutically acceptable salts
for the production of a therapeutical agent for curing or preventing amnesia.

## Patentansprüche (Patentansprüche für folgende(n) Vertragsstaat(en): DE, FR, GB)

1. Verwendung der folgenden Verbindung (I) 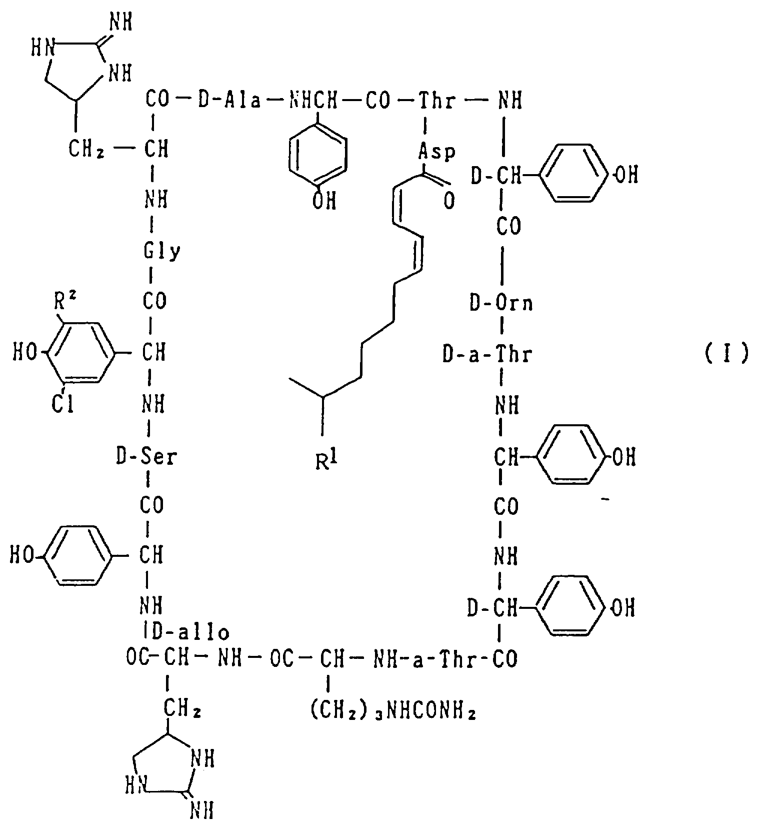 worin R¹ eine Alkylgruppe darstellt, R ein Halogenatom oder ein Wasserstoffatom ist, Ala einen Alanin-Rest bedeutet, Thr einen Threoninrest bedeutet, Orn einen Ornithinrest bedeutet, Ser einen Serinrest bedeutet und Gly einen Glycinrest bedeutet, D den Typ D bezeichnet und a = allo ein Stereoisomeres bezeichnet;
oder eines ihrer therapeutisch annehmbaren Salze für die Herstellung eines therapeutischen Mittels zur Inhibition einer Prolylendopeptidase-Aktivität in einem Patienten bzw. Subjekt.

2. Verwendung der Verbindung (I) gemäß Anspruch 1 oder eines ihrer therapeutisch annehmbaren Salze zur Herstellung eines therapeutischen Mittels zum Heilen oder Vorbeugen bei Amnesia.

## Revendications (Revendications pour l'(les) Etat(s) contractant(s) suivant(s): DE, FR, GB)

1. Utilisation du composé (I) suivant: 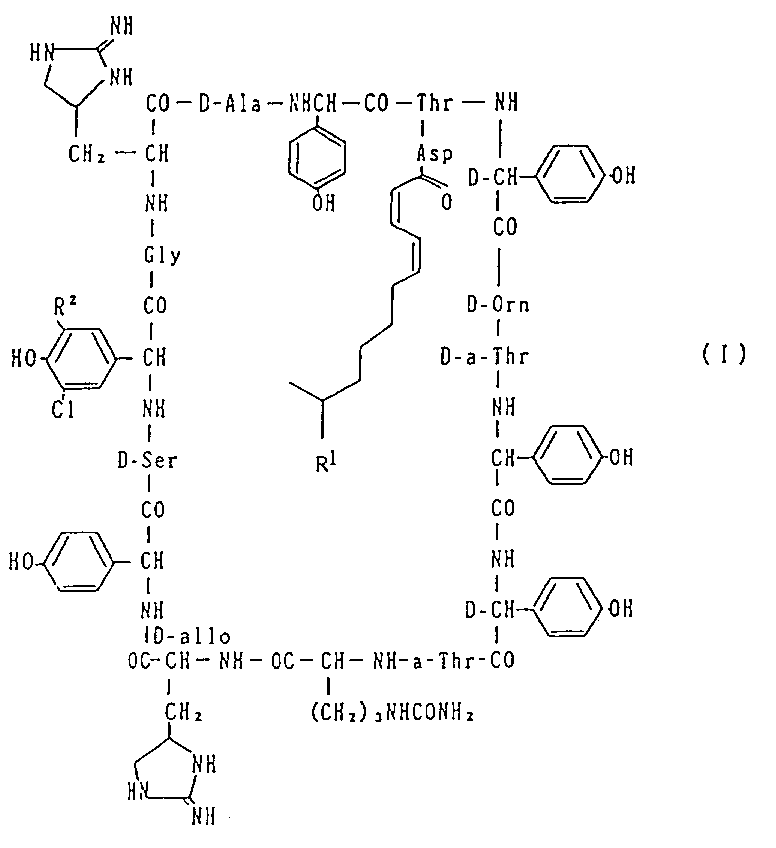 dans lequel R¹ représente un groupe alkyle, R est un atome d'halogène ou un atome d'hydrogène, Ala désigne un résidu alanine, Thr un résidu thréonine, Orn un résidu ornithine, Ser un résidu sérine et Gly un résidu glycine, D désigne le type D et a = allo désigne un stéréoisomère; ou d'un de ses sels acceptables en thérapeutique,
pour la production d'un agent thérapeutique pour l'inhibition de l'activité de la prolylendopeptidase chez un sujet.

2. Utilisation du composé (I) défini dans la revendication 1 ou d'un de ses sels acceptables en thérapeutique,
pour la production d'un agent thérapeutique pour guérir ou empêcher l'amnésie.
